# EUROPEAN PATENT APPLICATION

(11) **EP 3 932 443 A1**
(43) Date of publication of application: **05.01.2022**
(21) Application number: 20770432.1
(22) Date of filing: 07.02.2020
(51) Int. Cl.: A61M 1/02

(54) **TUBE WINDING BOBBIN AND BLOOD BAG SYSTEM**

(30) Priority: 14.03.2019 JP 2019047250
(71) Applicant: TERUMO Kabushiki Kaisha, Tokyo 151-0072 (JP)
(72) Inventor: TAKEDA, Norihiko, Fujinomiya-shi, Shizuoka 418-0004 (JP); SUZUKI, Takayuki, Tokyo 163-1450 (JP)
(74) Representative: TBK
(86) International application number: PCT/JP2020/004791
(87) International publication number: WO 2020/184017

(57) **Abstract**

A tube winding bobbin (84) is accommodated in a segment pocket (74) of a centrifugation transfer device (12) in which a blood bag system (10) is set. The blood bag system (10) is provided with a plurality of sealed tubes (28a) which extend for a predetermined length and which have an occluded lumen therein. The tube winding bobbin (84) has a winding part (86) around which the plurality of sealed tubes (28a) are wound, a pair of side parts (88) connected to both ends of the winding part (86), and an engagement part (92) engageable with a wall part (75) constituting the segment pocket (74).

## Description

### Technical Field

The present invention relates to a tube winding bobbin used when a blood bag system is set in a centrifugation transfer device, and a blood bag system to which the tube winding bobbin is applied.

### Background Art

During centrifugation of blood, a blood bag system including a blood bag that stores blood is set in a centrifugation transfer device. The centrifugation transfer device applies a centrifugal force to the set blood bag to separate the blood in the blood bag into a plurality of types of blood components, and transfers a predetermined blood component to another bag via a tube connected to the blood bag.

In this type of blood bag system, the blood of a donor is transferred to the blood bag via the tube and, then, a plurality of points of the tube connected to the blood bag is sealed. Thus, a segment tube is formed (see JP 2016-171967 A). The segment tube independently includes a plurality of sealed tubes in which blood is contained.

### Summary of Invention

The centrifugation transfer device includes a segment pocket that accommodates the segment tube such that the segment tube of the blood bag system does not interfere with the centrifugation transfer device during centrifugation. However, when the blood bag system is set in the centrifugation transfer device, the user may take time to accommodate the plurality of sealed tubes in the segment pocket, and the plurality of sealed tubes may stick out of the segment pocket depending on an accommodation state.

The present invention has been made in view of the above circumstances, and an object of the present invention is to provide a tube winding bobbin and a blood bag system capable of improving work efficiency by easily accommodating a plurality of sealed tubes and preventing the plurality of sealed tubes from sticking out.

In order to achieve the above object, a first aspect of the present invention is a tube winding bobbin attachable to a centrifugation transfer device in which a blood bag system is set, in which the blood bag system includes a tube member including a plurality of sealed tubes, the plurality of sealed tubes extending for a predetermined length and having a closed lumen therein, the tube winding bobbin includes a winding part around which the tube member is wound, and a pair of side parts coupled to both ends of the winding part and protruding in a thickness direction of the winding part, and the winding part has a length that is set such that the tube member is wound around the winding part in a state where the tube member is folded at a point of a coupling seal part that connects the plurality of sealed tubes to each other.

In order to achieve the above object, a second aspect of the present invention is a blood bag system including a first bag configured to accommodate blood, a second bag configured to accommodate a predetermined component in the blood, and a tube connecting the first bag and the second bag, in which the tube is provided with a tube winding bobbin around which the tube can be wound, and the tube winding bobbin includes a winding part around which the tube is wound, and a pair of side parts coupled to both ends of the winding part and protruding in a thickness direction of the winding part.

In the tube winding bobbin and the blood bag system, the plurality of sealed tubes can be accommodated in the pocket of the centrifugation transfer device in a state of being wound around the winding part of the tube winding bobbin. Thus, the plurality of sealed tubes is folded back without being disordered, and the plurality of sealed tubes can be smoothly inserted into the pocket.

### Brief Description of Drawings

Fig. 1 is an explanatory view illustrating an overall configuration of a blood bag system and a centrifugation transfer device according to an embodiment of the present invention.
Fig. 2 is a plan view of a unit set area of the centrifugation transfer device.
Fig. 3 is a perspective view of a tube winding bobbin around which a segment tube is wound and a segment pocket.
Fig. 4 is an explanatory view illustrating a state where the tube winding bobbin is provided in a relay tube of the blood bag system.
Fig. 5 is an explanatory view illustrating a state where the tube winding bobbin and the relay tube are attached according to another configuration example.
Fig. 6A is a sectional view taken along the line VI-VI in Fig. 3. Fig. 6B is a sectional view illustrating a state where the tube winding bobbin is accommodated in the segment pocket.
Fig. 7A is a perspective view of a tube winding bobbin according to a first modification. Fig. 7B is a perspective view of a tube winding bobbin according to a second modification.

### Description of Embodiments

Hereinafter, a preferred embodiment of the present invention will be described in detail with reference to the accompanying drawings.

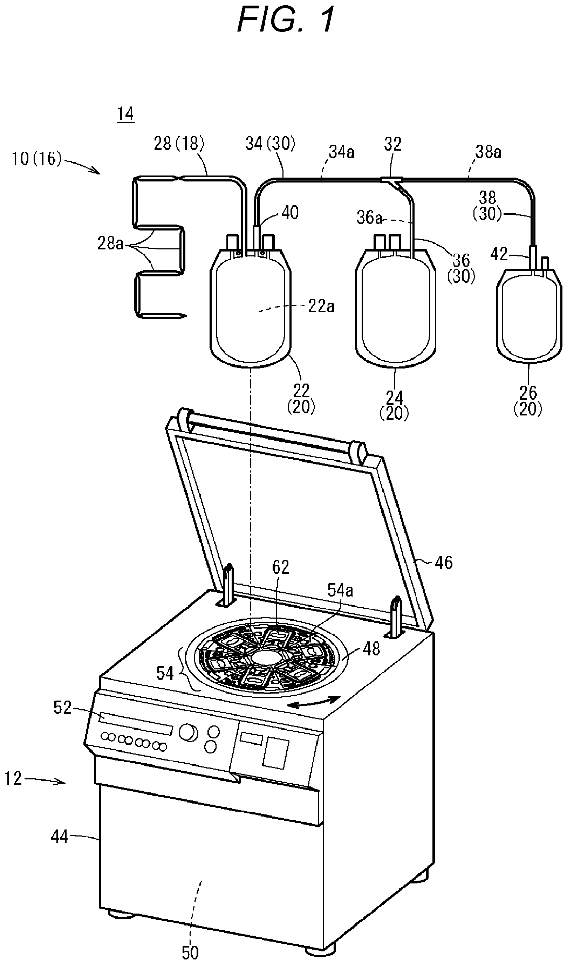

As illustrated in Figs. 1 to 3, a tube winding bobbin 84 according to an embodiment of the present invention is set in a centrifugation transfer device 12 together with a blood bag system 10 by a user such as a medical worker. The centrifugation transfer device 12 centrifuges blood in the blood bag system 10 to generate and store a plurality of types of blood components. Hereinafter, a configuration including the blood bag system 10 and the centrifugation transfer device 12 is referred to as a blood product manufacturing system 14.

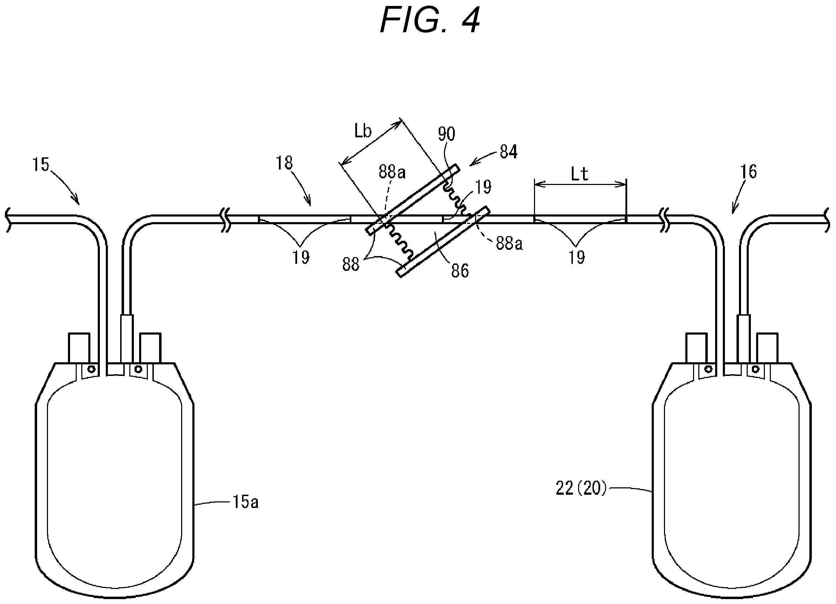

The blood bag system 10 includes a preprocessor 15 (see Fig. 4) used for blood collection before centrifugation, and a separation processor 16 that generates blood components by centrifugation and individually stores the blood components. The preprocessor 15 and the separation processor 16 are connected by a relay tube 18. The relay tube 18 connected to the preprocessor 15 is cut before centrifugation, and the separation processor 16 is in a state illustrated in Fig. 1, and is set in the centrifugation transfer device 12.

The preprocessor 15 of the blood bag system 10 collects whole blood from a donor and removes a predetermined blood component (for example, white blood cells) from the whole blood. Thus, the preprocessor 15 includes a blood sampling needle, a blood sampling bag 15a (first bag: see Fig. 4), an initial flow blood bag, a filter (for example, a white blood cell removal filter), and the like, and is configured by connecting each member by a plurality of tubes. The separation processor 16 is connected to downstream of the filter via the relay tube 18. Specifically, the preprocessor 15 stores initial blood of the whole blood of the donor collected through the blood sampling needle in the initial flow blood bag, and then stores residual blood in the blood sampling bag 15a. Furthermore, the preprocessor 15 removes white blood cells in the filter by causing the whole blood stored in the blood sampling bag 15a to flow to the filter, and transfers the removed blood (white blood cell removed blood) to the separation processor 16.

The separation processor 16 of the blood bag system 10 includes a plurality of bags 20 (a blood bag 22, a PPP bag 24, and a medical solution bag 26), and is configured by connecting the bags 20 by a plurality of tubes 30.

The blood bag 22 (second bag) is directly connected to the relay tube 18 connected to the preprocessor 15 in a state of the blood bag system 10 provided as a product. Thus, the blood bag 22 stores the removed blood transferred from the filter during blood collection. The blood bag 22 is set in the centrifugation transfer device 12, and a centrifugal force is applied by operation of the centrifugation transfer device 12. As a result, the removed blood in the blood bag 22 is centrifuged into blood components (platelet poor plasma (PPP) and red blood cells (RBC)) and the like having different specific gravities. Then, the blood bag 22 stores only the residual RBCs by transferring PPP during the operation of the centrifugation transfer device 12 after centrifugation.

The PPP is supplied from the blood bag 22, and the PPP bag 24 stores the PPP. The medical solution bag 26 stores in advance a red blood cell preservation solution such as MAP solution, SAGM solution, or OPTISOL.

Further, a segment tube 28 is formed in the separation processor 16 after the removed blood is transferred to the blood bag 22 and before the separation processor 16 is set in the centrifugation transfer device 12. The segment tube 28 includes a plurality of sealed tubes 28a having a predetermined length and having a closed lumen. Donor blood (removed blood) is present in the lumen of each sealed tube 28a. The relay tube 18 connecting the blood bag 22 and the preprocessor 15 (filter) is cut near the filter and is further sealed at predetermined intervals, and thus the plurality of sealed tubes 28a is formed so as to be continuous in series. The segment tubes 28 are cut by the user as necessary and used for checking blood and the like.

The plurality of tubes 30 of the separation processor 16 is branched into a plurality of tubes via a branch connector 32 (Y-shaped connector). Specifically, the plurality of tubes 30 includes a first tube 34 connecting the blood bag 22 and the branch connector 32, a second tube 36 connecting the PPP bag 24 and the branch connector 32, and a third tube 38 connecting the medical solution bag 26 and the branch connector 32. The first tube 34 includes a first flow path 34a, the second tube 36 includes a second flow path 36a, and the third tube 38 includes a third flow path 38a. The first to third flow paths 34a, 36a, and 38a communicate with each other via a flow path in the branch connector 32.

A breakable sealing member 40 (click tip) is provided at an end of the first tube 34 closer to the blood bag 22. Similarly, a breakable sealing member 42 is provided at an end of the third tube 38 closer to the medical solution bag 26. The sealing members 40 and 42 block the first flow path 34a and the third flow path 38a until breaking operation is performed, and prevent a liquid in the blood bag 22 and the medical solution bag 26 from being transferred to another bag 20.

The centrifugation transfer device 12 in which the separation processor 16 of the blood bag system 10 is set includes a box-shaped base body 44, a lid 46 capable of opening and closing an upper surface of the base body 44, and a centrifugal drum 48 provided in the base body 44. Further, the base body 44 of the centrifugation transfer device 12 is provided with a motor (not illustrated) that rotates the centrifugal drum 48, a controller 50 that controls the operation of the centrifugation transfer device 12, and an operation display 52 for the user to confirm and operate.

The centrifugal drum 48 has a plurality of (six) unit set areas 54 in which the separation processor 16 can be set. A height of one unit set area 54 is larger than a length of the bag 20 in a long-length direction, and is set in a range of 60° with respect to a rotation center of the centrifugal drum 48. That is, the six unit set areas 54 are arranged side by side without a gap along a circumferential direction to constitute the centrifugal drum 48 as an annular structure.

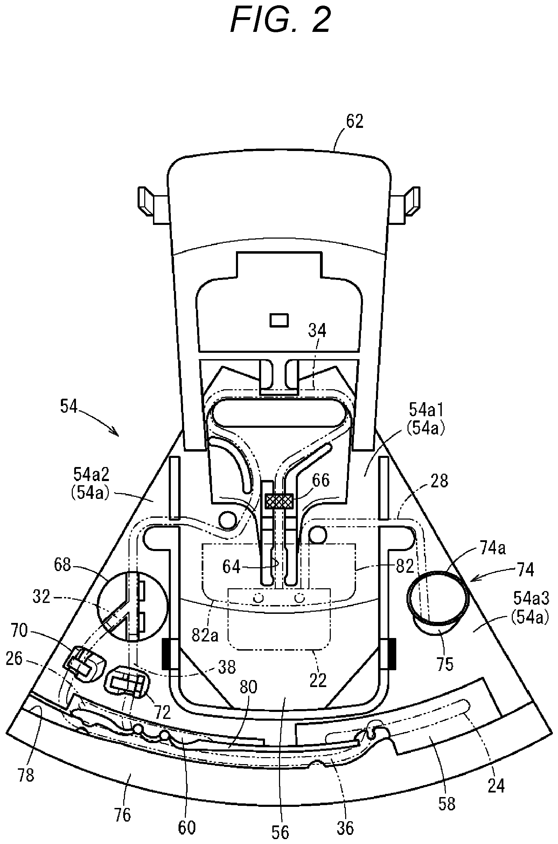

As illustrated in Figs. 1 and 2, the unit set areas 54 apply a centrifugal force to the blood bag 22 as the centrifugal drum 48 rotates. Specifically, each of the unit set areas 54 includes a blood bag pocket 56 accommodating the blood bag 22, a PPP bag pocket 58 accommodating the PPP bag 24, and a medical solution bag pocket 60 accommodating the medical solution bag 26 radially outside of the centrifugal drum 48.

The blood bag pocket 56 is provided at a circumferential center of the unit set area 54 and has a larger volume than the PPP bag pocket 58 and the medical solution bag pocket 60. The PPP bag pocket 58 and the medical solution bag pocket 60 are provided side by side in the circumferential direction radially outside of the blood bag pocket 56.

An upper surface 54a of the unit set area 54 is configured to arrange and hold the plurality of tubes 30 of the blood bag system 10. A part of the first tube 34 and a part of the segment tube 28 are disposed in a central region 54a1 (first region) radially inside of the blood bag pocket 56 in the upper surface 54a. The central region 54a1 is provided with a top cover 62 that opens and closes an opening of the blood bag pocket 56. Further, a sensor 66 (optical sensor) that detects a part of the breaker 64 breaking the sealing member 40 and a state of blood flowing through the first tube 34 is provided at an arrangement position of the first tube 34 closed by the top cover 62.

A part of the first tube 34, a part of the branch connector 32, a part of the second tube 36, and a part of the third tube 38 that have passed through the central region 54a1 are disposed in a left region 54a2 (second region) of the upper surface 54a. The left region 54a2 is provided with a holder 68 that holds the branch connector 32, a PPP clamp 70 that opens and closes the second flow path 36a of the second tube 36, and a medical solution clamp 72 that opens and closes the third flow path 38a of the third tube 38.

A part of the segment tube 28 that passes through the central region 54a1 is disposed in a right region 54a3 (third region) of the upper surface 54a. The right region 54a3 is provided with a segment pocket 74 that accommodates the plurality of sealed tubes 28a of the segment tube 28.

Furthermore, a tube holder 76 that protruding upward of the upper surface 54a is provided radially outside of the PPP bag pocket 58 and the medical solution bag pocket 60 of the unit set area 54. The tube holder 76 has an outer wall 78 continuous with an outer peripheral edge of the unit set area 54 and an inner wall 80 protruding inward of the outer wall 78 from the outer peripheral edge, and the second tube 36 is disposed between the outer wall 78 and the inner wall 80. The inner wall 80 extends from near the PPP clamp 70 in the left region 54a2 (left side of the medical solution bag pocket 60) to a circumferential intermediate position of the PPP bag pocket 58, and guides the second tube 36 to the PPP bag pocket 58.

Further, the unit set area 54 includes a slider 82 (pusher) that presses the blood bag 22 after centrifugation radially inside of the blood bag pocket 56. A surface of the slider 82 facing the blood bag 22 is formed as an inclined surface 82a. The slider 82 moves forward and backward along a radial direction of the centrifugal drum 48 under control of the controller 50 (see Fig. 1).

In the unit set area 54, the user accommodates the blood bag 22 storing the removed blood in the blood bag pocket 56, the empty PPP bag 24 in the PPP bag pocket 58, and the medical solution bag 26 storing the medical solution in the medical solution bag pocket 60. Then, the unit set area 54 centrifuges the removed blood in the blood bag 22 by the rotation of the centrifugal drum 48, advances the slider 82 after the centrifugation, and presses the blood bag 22. As a result, PPP generated by centrifugation in the blood bag 22 is transferred to the PPP bag 24, and the RBC remains in the blood bag 22 after the transfer of PPP. Thereafter, the blood bag system 10 is taken out from the centrifugation transfer device 12 by the user, and the medical solution bag 26 is hung on a stand (not illustrated). As a result, the red blood cell preservation solution is supplied from the medical solution bag 26 to the blood bag 22, and the blood bag 22 is storing RBC (blood product) including a medical solution.

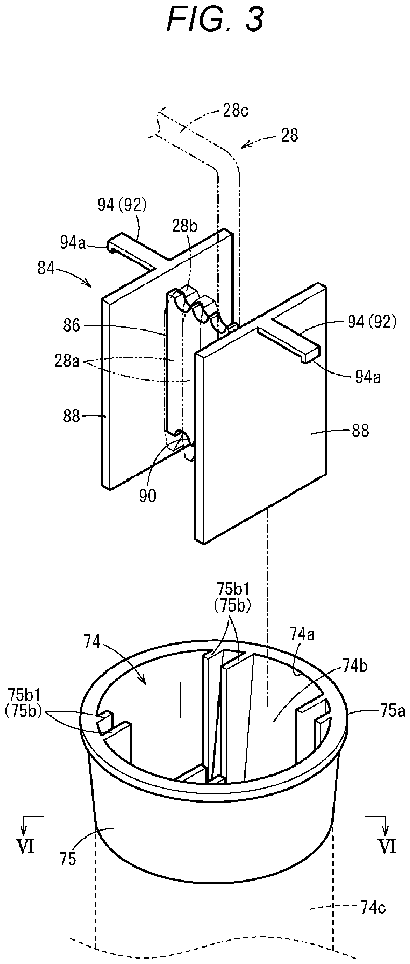

Next, a configuration of the tube winding bobbin 84 according to the present embodiment will be described with reference to Fig. 3.

The tube winding bobbin 84 is a member provided for the segment tube 28 of the blood bag system 10 to be wound around, and is accommodated in the segment pocket 74 of the centrifugation transfer device 12 (unit set area 54) in a state where the segment tube 28 is wound around. That is, the tube winding bobbin 84 is integrally accommodated with the segment tube 28 in the segment pocket 74, and this facilitates setting of the segment tube 28 in the centrifugation transfer device 12.

The segment tube 28 has a plurality of (for example, seven to eight) sealed tubes 28a as described above. A coupling seal part 28b that is sealed by crushing the relay tube 18 is formed between the adjacent sealed tubes 28a. An extending length of each of the sealed tubes 28a (the distance of an intermediate position between the adjacent coupling seal parts 28b) is not limited, but is a dimension of, for example, about 6 cm to 8 cm.

Further, the segment tube 28 includes an elongated coupling tube 28c that connects the plurality of sealed tubes 28a and the blood bag 22. In a state where the blood bag system 10 is set in the centrifugation transfer device 12, the coupling tube 28c extends on the upper surface 54a (the central region 54a1 and the right region 54a3) of the unit set area 54 and allows the plurality of sealed tubes 28a to reach the segment pocket 74.

The tube winding bobbin 84 includes a winding part 86 around which the plurality of sealed tubes 28a is wound, and a pair of side parts 88 coupled to both ends of the winding part 86 in an extending direction. The winding part 86 and the pair of side parts 88 are integrally molded to be continuous in series.

The winding part 86 has a rectangular plate shape, and the side parts 88 are coupled to the winding part 86 along a pair of long sides continuous to both ends in a short-length direction. The extending length along a long-length direction of the winding part 86 is slightly shorter than the extending length of the plurality of sealed tubes 28a. For example, an extending length Lb (see Fig. 4) of the winding part 86 in the long-length direction is set in a range of 5 cm to 7.5 cm.

A plate thickness of the winding part 86 is to be set to an appropriate thickness corresponding to ends of the adjacent sealed tubes 28a and the coupling seal parts 28b. For example, a thickness of the winding part 86 is set to be in a range of about 5 mm to 20 mm.

Further, a plurality of guide notches 90 that guides arrangement of the segment tube 28 is provided on a pair of short sides (sides at both ends in the long-length direction) of the winding part 86. The plurality of guide notches 90 has a recessed shape (arc shape) in which the short sides are recessed inward, and a plurality of guide notches is arranged side by side along the short sides. A dimension along the short side of each guide notch 90 is set to match an outer diameter of the sealed tube 28a.

The winding part 86 formed as described above can be wound around the winding part 86 while the plurality of sealed tubes 28a is folded at the coupling seal part 28b. That is, a body of each sealed tube 28a extends on one plate surface of the winding part 86, and both ends continuous with the body protrude from both ends of the winding part 86 in the long-length direction. The coupling seal part 28b connected to both ends easily reverses an extending direction of the segment tube 28 while being guided by an arbitrary guide notch 90, and the sealed tube 28a extends along the other plate surface of the winding part 86.

Here, the blood bag system 10 may be provided as a product as illustrated in Fig. 4, including the tube winding bobbin 84. Specifically, the tube winding bobbin 84 is provided on the relay tube 18 connecting the blood sampling bag 15a and the blood bag 22 storing blood, downstream of the filter (not illustrated) of the preprocessor 15. For example, the tube winding bobbin 84 is provided together with the blood bag system 10 in a state where each of the pair of side parts 88 has an attachment hole 88a and the relay tube 18 is inserted through the pair of attachment hole 88a.

The pair of attachment holes 88a is provided on one end of the pair of side parts 88 in one long-length direction and on the other end of the pair of side parts 88 in the other long-length direction. Each attachment hole 88a has an inner diameter having a margin to some extent with respect to an outer diameter of the relay tube 18. That is, the tube winding bobbin 84 is displaceable along an extending direction of the relay tube 18. As described above, the relay tube 18 is cut before the centrifugation transfer device 12 is set, and thus the tube winding bobbin 84 can be detached from a cut point of the relay tube 18. Therefore, in the tube winding bobbin 84, the plurality of sealed tubes 28a can be satisfactorily wound around the winding part 86 when the segment tube 28 is wound.

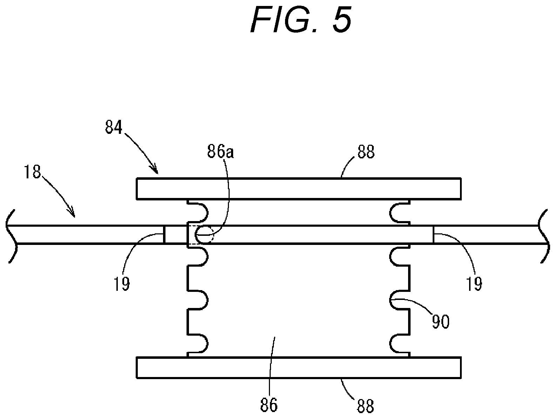

A method of providing the tube winding bobbin 84 on the relay tube 18 is not limited, and for example, as illustrated in Fig. 5, one attachment hole 86a may be provided on one side of the plate-shaped winding part 86 in the long-length direction. The attachment hole 86a is formed in a crest between two adjacent guide notches 90 near one side part 88. The relay tube 18 is provided as a product together with the blood bag system 10 while being movably inserted into the attachment hole 86a. In the relay tube 18 passing through the attachment hole 86a of the winding part 86, the plurality of sealed tubes 28a can be wound around the winding part 86 with the relay tube 18 passing through the attachment hole 86a after the plurality of sealed tubes 28a is formed by a sealer (not illustrated).

Note that the tube winding bobbin 84 may be fixed to the relay tube 18 when the blood bag system 10 is provided as a product. For example, the tube winding bobbin 84 is fixed to one end (for example, a vicinity of a cut point between the preprocessor 15 and the separation processor 16) of the plurality of sealed tubes 28a to be formed, and thus the plurality of sealed tubes 28a can be wound with this fixed position as a base point.

In Fig. 4 again, the blood bag system 10 is preferably provided with a plurality of indicators 19 that guides formation of the sealed tube 28a in accordance with the tube winding bobbin 84. For example, each indicator 19 is formed as a linear scale that goes around a surface of the relay tube 18 in a circumferential direction.

An interval Lt between the adjacent indicators 19 of the indicators 19 is set to be slightly longer than the extending length Lb in the long-length direction of the winding part 86 of the tube winding bobbin 84. Each of the indicators 19 guides a sealed position by the user when the sealed tube 28a is formed to indirectly lead the formation of each sealed tube 28a having a length corresponding to the winding part 86. It is therefore preferable to set the interval Lt between the adjacent indicators 19 appropriately in consideration of a sealing range of the relay tube 18.

In Fig. 3 again, the pair of side parts 88 of the tube winding bobbin 84 is a plate body extending along the long side of the winding part 86, and has a rectangular shape whose length in the long-length direction is longer than a length in the long-length direction of the winding part 86. A short-length direction of the pair of side parts 88 protrudes in a direction orthogonal to a surface direction of the winding part 86. That is, the tube winding bobbin 84 has an H-shape in plan view. In the tube winding bobbin 84, the pair of side parts 88 protrudes from both ends in the long-length direction and the plate thickness of the winding part 86. This protrusion prevents the segment tube 28 wound around the winding part 86 from coming off.

The tube winding bobbin 84 includes an engagement part 92 at one end of the pair of side parts 88 in the long-length direction. The engagement part 92 engages the tube winding bobbin 84 with a wall 75 constituting the segment pocket 74. In the present embodiment, the engagement part 92 includes a pair of claws 94 that can be caught by an edge 75a constituting an opening 74a of the segment pocket 74.

The pair of claws 94 extends outward in a width direction (direction orthogonal to a plate surface of the side part 88) from short sides of the pair of side parts 88. The pair of claws 94 is coupled to an intermediate position of the short side of the side part 88, and is provided at positions opposite to each other across the winding part 86. Extending ends of the pair of claws 94 protrude outward from the edge 74a of the segment pocket 75.

A barb 94a that is curved (or bent) with respect to an extending direction of the pair of claws 94 is formed at each extending end of the pair of claws 94. For example, the barb 94a is curved toward the side of the pair of side parts 88 (a lower side in Fig. 3) and is configured to be caught by the edge 75a of the segment pocket 74, the edge 75a having a circular cross section.

On the other hand, the segment pocket 74 of the centrifugation transfer device 12 (unit set area 54) has the opening 74a formed to be larger than a planar shape (H-shape) of the tube winding bobbin 84, and a region near the opening 74a formed as a tapered space 74b that narrows with increase in depth in an insertion direction. Further, a cylindrical space 74c extending with a constant inner diameter is formed in a deeper part than the region near the opening 74a (tapered space 74b) of the segment pocket 74.

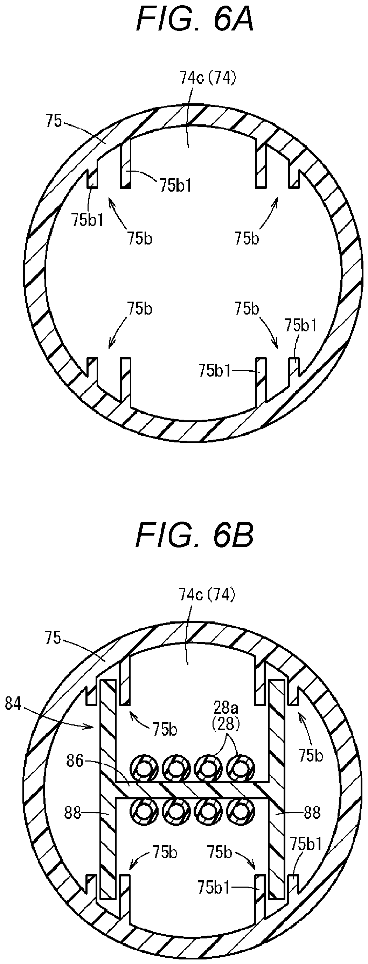

As illustrated in Fig. 6A, the wall 75 of the segment pocket 74 constituting the cylindrical space 74c has a plurality of (four in Fig. 6A) guides 75b protruding inward. The plurality of guides 75b guides the insertion of the pair of side parts 88 of the tube winding bobbin 84. Therefore, the plurality of guides 75b is constituted by a first set and a second set protruding in parallel from an inner surface (wall 75), and the first set and the second set face each other across the segment pocket 74.

The plurality of guides 75b includes a pair of ridges 75b1 protruding inward from the wall 75 (inner surface), and long sides of the side parts 88 are disposed between the pair of ridges 75b1. The pair of ridges 75b1 extends along an axial direction of the cylindrical space 74c and guides the insertion of the side parts 88.

The tube winding bobbin 84 and the blood bag system 10 according to the present embodiment are basically configured as described above, and the operation thereof will be described below.

As described above, the blood bag system 10 stores, in the blood bag 22, the removed blood obtained by removing a predetermined component (white blood cells) from the whole blood of the donor with use of the preprocessor 15 during blood collection by the user (medical worker). In a storage space 22a, an appropriate volume of blood in accordance with the donor is stored.

Thereafter, the user separates the separation processor 16 of the blood bag system 10 from the preprocessor 15 in order to centrifuge the removed blood. At this time, the cut relay tube 18 is sealed at a plurality of points at predetermined intervals by a sealer (not illustrated) to form the segment tube 28 having the plurality of sealed tubes 28a. As illustrated in Fig. 4, in the blood bag system 10, the plurality of indicators 19 is indicated on the relay tube 18, and thus the user can be guided to seal points of the indicators 19. After the segment tube 28 is formed, the user sets the separation processor 16 in the centrifugation transfer device 12.

As illustrated in Fig. 2, the blood bag 22 is accommodated in the blood bag pocket 56, and the tube 30 (the branch connector 32 and the first to third tubes 34, 36, and 38) is disposed at an appropriate position along the upper surface 54a. Further, the PPP bag 24 is accommodated in the PPP bag pocket 58, and the medical solution bag 26 is accommodated in the medical solution bag pocket 60.

In the segment tube 28, the coupling tube 28c extends from the central region 54a1 of the upper surface 54a toward the right region 54a3, and the coupling tube 28c further extends outward in a centrifugal direction in the right region 54a3. The segment pocket 74 is attached to the right region 74a3 with the opening 54a slightly inclined inward in the centrifugal direction to facilitate insertion of the segment tube 28.

In the segment tube 28, the plurality of sealed tubes 28a is wound around the tube winding bobbin 84 by the user. The plurality of sealed tubes 28a arranged side by side in the extending direction is wound around the tube winding bobbin 84 while extending along one surface and the other surface, alternately, of the winding part 86. As a result, the plurality of sealed tubes 28a is easily disposed inside of the long sides of the pair of side parts 88.

The tube winding bobbin 84 is accommodated in the segment pocket 74 together with the wound sealed tube 28a. At this time, as illustrated in Fig. 6B, the user can insert the long sides of the pair of side parts 88 into the plurality of guides 75b (parts between the pair of ridges 75b1) provided on the wall 75 constituting the segment pocket 74 in a state where a position of the tube winding bobbin 84 is defined.

The tube winding bobbin 84 maintains a winding state of the plurality of sealed tubes 28a even when accommodated in the segment pocket 74. That is, even if the sealed tube 28a moves so as to spread (separate from the winding part 86) in the segment pocket 74, the sealed tube 28a cannot be detached from a narrow space between the winding part 86 and the wall 75. Therefore, the tube winding bobbin 84 can prevent the plurality of sealed tubes 28a from coming off from the segment pocket 74.

Further, in the tube winding bobbin 84, the pair of claws 94 constituting the engagement part 92 is caught by the edge 75a as the segment pocket 74 is inserted. This also suppresses detachment of the tube winding bobbin 84 from the wall 75.

After the blood bag system 10 (segment tube 28) is set, the centrifugation transfer device 12 centrifuges the removed blood in the blood bag 22 into blood components (PPP, RBC, and the like) having different specific gravities by rotating the centrifugal drum 48 under the control of the controller 50. As described above, the segment tube 28 continues to be well accommodated in the segment pocket 74, and does not interfere with a periphery during centrifugation. The blood in the plurality of sealed tubes 28a extending along the centrifugal direction is separated into blood components by centrifugation.

After centrifugation, the centrifugation transfer device 12 presses the blood bag 22 with the slider 82. As a result, PPP having a light specific gravity flows out from the blood bag 22, and the PPP flows through the first tube 34, the branch connector 32, and the second tube 36 in that order and flows into the PPP bag 24. Further, when the PPP is transferred from the blood bag 22 to the PPP bag 24, the centrifugation transfer device 12 retracts the slider 82 such that the blood bag 22 can be taken out from the blood bag pocket 56.

Thereafter, the user removes the blood bag system 10 from the centrifugation transfer device 12. At this time, the engagement of the engagement part 92 of the tube winding bobbin 84 is released, and the segment tube 28 is also taken out together with the tube winding bobbin 84. Upon release of winding of the sealed tube 28a, the tube winding bobbin 84 is reused for the next centrifugation. Further, the user suspends the medical solution bag 26 on the stand to supply the red blood cell preservation solution to the blood bag 22. As a result, the RBC including the red blood cell preservation solution is stored in the blood bag 22.

Note that the present invention is not limited to the above embodiment, and various modifications can be made in accordance with the gist of the invention. For example, in the above embodiment, the guide 75b is provided inside the segment pocket 74, but the guide 75b does not have to be provided. Further, instead of guiding the pair of side parts 88 of the tube winding bobbin 84, the guide 75b can engage with the pair of side parts 88. Alternatively, the tube winding bobbin 84 may include the winding part 86 having a substantially tubular shape.

Hereinafter, some other modifications of the present invention will be described. Note that, in the following description, the same reference signs are given to constituent elements having the same configurations or the same functions as constituent elements of the above embodiment, and a detailed description thereof will be omitted.

### First Modification

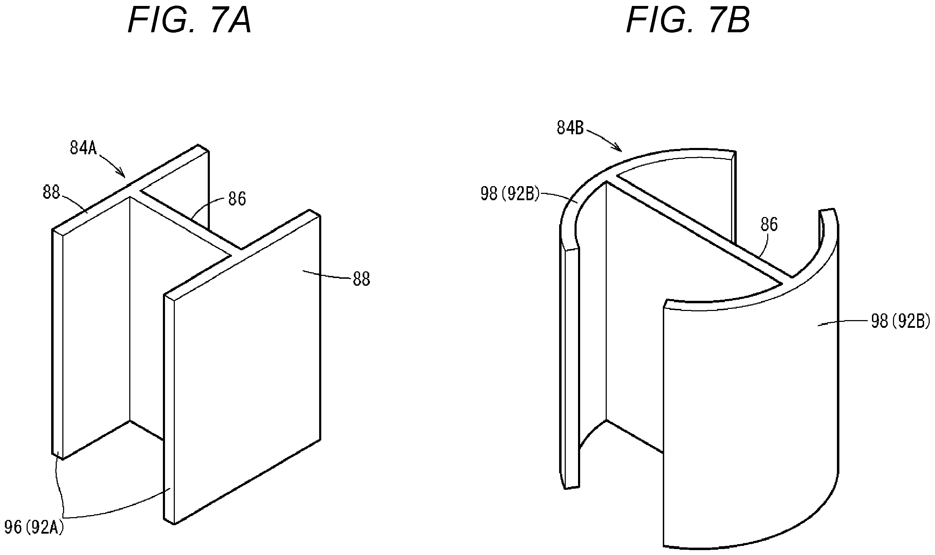

In a tube winding bobbin 84A according to a first modification illustrated in Fig. 7A, the lengths in the long-length direction of the winding part 86 and the pair of side parts 88 are set to the same dimension, and the tube winding bobbin 84A has an H-shape in plan view. The tube winding bobbin 84A is configured such that the pair of long sides (hereinafter, referred to as protruding sides 96) of each side part 88 is fittable to the wall 75 of the segment pocket 74. That is, the protruding sides 96 cooperate with the wall 75 to serve as an engagement part 92A that prevents detachment of the tube winding bobbin 84A.

The tube winding bobbin 84A configured as described above can also obtain similar effects to effects of the above embodiment. The manufacturing can be facilitated and the manufacturing cost can be reduced particularly by simplified configurations of the tube winding bobbin 84A and the centrifugation transfer device 12 (segment pocket 74) .

### Second Modification

In a tube winding bobbin 84B according to a second modification illustrated in Fig. 7B, a pair of side parts 98 coupled to the winding part 86 has an arc shape in plan view. A curvature of the pair of side parts 98 is set to be the same as a curvature of an inner surface of the segment pocket 74, and a diameter of a virtual circle formed by the pair of side parts 98 matches an inner diameter of the segment pocket 74.

That is, the tube winding bobbin 84B has a configuration in which an outer surface of the pair of side parts 98 is in surface contact with and fitted to the wall 75 of the segment pocket 74, and the pair of side parts 98 functions as an engagement part 92B that prevents detachment of the tube winding bobbin 84B. The tube winding bobbin 84B configured as described above can also obtain similar effects to effects of the above embodiment. In particular, in the pair of side parts 98, the sealed tube 28a wound around the winding part 86 can be disposed on an inner surface of the arc shape. This can suppress disengagement of the sealed tube 28a before insertion into the segment pocket 74 or the like.

The engagement parts 92, 92A, and 92B described above may be singly provided, or may be configured by combining a plurality of types, and thus the tube winding bobbins 84, 84A, and 84B can be firmly fixed to the segment pocket 74.

Technical ideas and effects that can be recognized from the above embodiment will be described below.

In the tube winding bobbin 84, the plurality of sealed tubes 28a can be accommodated in the pocket (segment pocket 74) of the centrifugation transfer device 12 in a state of being wound around the winding part 86 of the tube winding bobbin 84. Thus, the plurality of sealed tubes 28a is folded back without being disordered, and the plurality of sealed tubes 28a can be smoothly inserted into the segment pocket 74.

Further, the winding part 86 is as long as the extending length of the sealed tube 28a or shorter than the extending length of the sealed tube 28a. Thus, in the tube winding bobbin 84, the sealed tube 28a can be extended along one plate surface of the winding part 86 and folded back at a boundary between the sealed tubes 28a, and the plurality of sealed tubes 28a can be disposed stably.

Further, the winding part 86 has a plate shape or a substantially tubular shape. As a result, for example, the winding part 86 can arrange the plurality of sealed tubes 28a to be easily folded back. In a state of being accommodated in the segment pocket 74, the plurality of sealed tubes 28a can satisfactorily receive a centrifugal force along the extending direction of the plurality of sealed tubes 28a.

Further, the winding part 86 includes the guide notch 90 that guides arrangement of the plurality of sealed tubes 28a or the coupling seal part 28b connecting the plurality of sealed tubes 28a to each other. Thus, in the tube winding bobbin 84, the plurality of sealed tubes 28a can be neatly wound around the winding part 86, and the tube winding bobbin 84 can be more smoothly inserted into the segment pocket 74 together with the plurality of sealed tubes 28a.

Further, the engagement parts 92, 92A, and 92B engageable with the wall 75 constituting the pocket (segment pocket 74) are provided. As a result, the tube winding bobbin 84 is prevented from being detached from the segment pocket 74 in a state of being accommodated in the segment pocket 74.

Further, the engagement part 92 includes the claws 94 that can be caught by the edge 75a constituting the opening 74a of the pocket (segment pocket 74). Thus, in the tube winding bobbin 84, the claw 94 can easily engage with the edge 74a of the segment pocket 75. The claw 94 engaged with the edge 75a enables the tube winding bobbin 84 to be easily taken out (disengaged) from the segment pocket 74.

Further, the engagement parts 92A may include the protruding sides 96 of the pair of side parts 88 that is fittable to the wall 75 constituting the pocket (segment pocket 74). As a result, the tube winding bobbin 84A is engaged with the segment pocket 74 only by the operation of inserting the tube winding bobbin 84 into the segment pocket 74. Further, the tube winding bobbin 84 itself has a simple configuration, and thus allows the plurality of sealed tubes 28a to be smoothly wound.

Further, the engagement part 92B includes the outer surface of the pair of side parts 98 that can be in surface contact with the wall 75 constituting the pocket (segment pocket 74). Thus, the tube winding bobbin 84B is firmly engaged with the segment pocket 74 only by the operation of being inserted into the segment pocket 74.

Further, the blood bag system 10 includes the first bag (blood sampling bag 15a) configured to accommodate blood, the second bag (blood bag 22) configured to accommodate a predetermined component in the blood, and the tube (relay tube 18) connecting the first bag and the second bag, in which the tube is provided with the tube winding bobbin 84 around which the tube can be wound, and the tube winding bobbin 84 includes the winding part around 86 which the tube is wound, and a pair of side parts 88 coupled to both ends of the winding part 86 and protruding in a thickness direction of the winding part 86. Thus, the blood bag system 10 can be provided together with the tube winding bobbin 84. By using the tube winding bobbin 84, the plurality of sealed tubes 28a can be easily accommodated, work efficiency can be improved, and the plurality of sealed tubes 28a can be prevented from sticking out.

The indicators 19 are provided on the surface of the tube (relay tube 18) at predetermined intervals, and the length of the winding part 86 is set to be equal to or less than a tube length between the adjacent indicators 19. Thus, the plurality of sealed tubes 28a can be easily formed along the indicators 19, and can be easily wound around the tube winding bobbin 84.

The tube winding bobbin 84 has at least one attachment hole 86a or 88a into which the tube (relay tube 18) is inserted and that allows the tube winding bobbin 84 to move along the extending direction of the tube. As a result, the blood bag system 10 allows the tube winding bobbin 84 to be detached from the relay tube 18 at time of use and allows the plurality of sealed tubes 28a to be satisfactorily wound around the tube winding bobbin 84.

## Claims

1. A tube winding bobbin attachable to a centrifugation transfer device in which a blood bag system is set, wherein
the blood bag system includes a tube member including a plurality of sealed tubes, the plurality of sealed tubes extending for a predetermined length and having a closed lumen therein,
the tube winding bobbin includes
a winding part around which the tube member is wound, and
a pair of side parts coupled to both ends of the winding part and protruding in a thickness direction of the winding part, and
the winding part has a length that is set such that the tube member is wound around the winding part in a state where the tube member is folded at a point of a coupling seal part that connects the plurality of sealed tubes to each other.

2. The tube winding bobbin according to claim 1, wherein
the winding part has a length that is same as an extension length of the plurality of sealed tubes or shorter than the extending length of the plurality of sealed tubes.

3. The tube winding bobbin according to claim 1 or 2, wherein
the winding part has a plate shape or a substantially tubular shape.

4. The tube winding bobbin according to any one of claims 1 to 3,
wherein the winding part includes the coupling seal part that connects the plurality of sealed tubes to each other or a guide notch that guides arrangement of the plurality of sealed tubes or.

5. The tube winding bobbin according to any one of claims 1 to 4, comprising
an engagement part that is engageable with a wall constituting a pocket of the centrifugation transfer device.

6. The tube winding bobbin according to claim 5, wherein
the engagement part includes a claw that can be caught by an edge constituting an opening of the pocket.

7. The tube winding bobbin according to claim 5 or 6, wherein
the engagement part includes protruding sides of the pair of side parts, the protruding sides being fittable to the wall constituting the pocket.

8. The tube winding bobbin according to any one of claims 5 to 7, wherein
the engagement part includes an outer surface of the pair of side parts configured to be in surface contact with the wall constituting the pocket.

9. A blood bag system comprising:
a first bag configured to accommodate blood;
a second bag configured to accommodate a predetermined component in the blood; and
a tube connecting the first bag and the second bag, wherein
the tube is provided with a tube winding bobbin around which the tube can be wound, and
the tube winding bobbin includes
a winding part around which the tube is wound, and
a pair of side parts coupled to both ends of the winding part and protruding in a thickness direction of the winding part.

10. The blood bag system according to claim 9, wherein
the tube is provided with indicators on a surface of the tube at predetermined intervals, and
the winding part has a length that is set to be equal to or less than a tube length between indicators adjacent each other.

11. The blood bag system according to claim 9 or 10, wherein the tube winding bobbin includes at least one attachment hole into which the tube is inserted and that allows the tube winding bobbin to move along an extending direction of the tube.
